(19) 

Europäisches
Patentamt
European
Patent Office
Office européen
des brevets

(11) **EP 4 148 037 A1**

(12) **EUROPEAN PATENT APPLICATION**
published in accordance with Art. 153(4) EPC

(43) Date of publication:
**15.03.2023 Bulletin 2023/11**

(21) Application number: **22739730.4**

(22) Date of filing: **13.01.2022**

(51) International Patent Classification (IPC):
$C07C\ 51/43^{(2006.01)}$    $C07C\ 51/50^{(2006.01)}$
$C07C\ 59/01^{(2006.01)}$    $C12P\ 7/42^{(2006.01)}$
$C12N\ 15/70^{(2006.01)}$    $C12N\ 9/88^{(2006.01)}$
$C12N\ 9/02^{(2006.01)}$    $C12N\ 9/10^{(2006.01)}$

(52) Cooperative Patent Classification (CPC):
**C07C 51/43; C07C 51/50; C07C 59/01;
C12N 9/0004; C12N 9/10; C12N 9/88; C12N 15/70;
C12P 7/42**

(86) International application number:
**PCT/KR2022/000673**

(87) International publication number:
**WO 2022/154537 (21.07.2022 Gazette 2022/29)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **15.01.2021 KR 20210006246
08.09.2021 KR 20210119997**

(71) Applicant: **Lg Chem, Ltd.
Seoul 07336 (KR)**

(72) Inventors:
• **JUNG, Woochul
Daejeon 34122 (KR)**

• **HUH, In Young
Daejeon 34122 (KR)**
• **LEE, Kyung Muk
Daejeon 34122 (KR)**
• **KANG, Donggyun
Daejeon 34122 (KR)**
• **KIM, Jae Hyung
Daejeon 34122 (KR)**

(74) Representative: **Goddar, Heinz J.
Boehmert & Boehmert
Anwaltspartnerschaft mbB
Pettenkoferstrasse 22
80336 München (DE)**

(54) **CRYSTALS OF 3-HYDROXYPROPIONATE AND PROCESS OF RECOVERING
3-HYDROXYPROPIONIC ACID**

(57)    Provided is a process of recovering 3-hydroxy-propionic acid and a crystal of 3-hydroxypropionate, wherein the process includes the steps of: forming a crystal of 3-hydroxypropionate in a concentrate containing 3-hydroxypropionic acid in an amount of 300 g/L or more of in the presence of an alkali metal salt; and separating the crystals of 3-hydroxypropionate from the concentrate and converting into 3-hydroxypropionic acid.

**FIG. 1**

**EP 4 148 037 A1**

**Description**

CROSS-REFERENCE TO RELATED APPLICATION(S)
This application is a National Stage Application of International Application No. PCT/KR2022/000673 filed on January 13, 2022, which claims the benefits of Korean Patent Applications No. 10-2021-0006246 filed on January 15, 2021 and No. 10-2021-0119997 filed on September 8, 2021 with the Korean Intellectual Property Office, the disclosure of which is incorporated herein by reference in its entirety.

**FIELD OF THE INVENTION**

The present disclosure relates to a crystal of 3-hydroxypropionate and a process of recovering 3-hydroxypropionic acid (3HP). More particularly, it relates to a process of recovering 3-hydroxypropionic acid with crystallization of 3-hydroxypropionate and a crystal of 3-hydroxypropionate formed in this process.

**BACKGROUND**

3-hydroxypropionic acid (3HP) is a platform compound which can be converted into various chemicals such as acrylic acid, methyl acrylate, acrylamide, etc. 3-hydroxypropionic acid has been actively studied in academic and industrial circles since it was selected as one of top 12 value-added bio-chemicals by the U.S. Department of Energy (DOE) in 2004.
The production of 3-hydroxypropionic acid is largely performed by two methods: a chemical method and a biological method. However, the chemical method has been criticized that an initial material is expensive and is not eco-friendly because of generating toxic substances during a production process, etc., so an environmentally friendly biological method is in the spotlight.
In the preparation of organic acids by microbial fermentation, other by-products in addition to organic acids such as 3-hydroxypropionic acid, etc., are also generated in the process of fermenting microorganisms, and thus a process of extracting and separating the organic acids from a fermented solution is required. Electrodialysis, reverse osmosis, solution-organic solvent reaction extraction containing organic acids, and the like are used as a method for extracting and separating organic acids from microbial fermented solution. In particular, a reverse extraction method with sodium hydroxide (NaOH) is widely used due to its high yield. However, these methods result in products in the form of an organic acid salt, and thus have a disadvantage of requiring a process for converting the products into an organic acid and having low purity.
Unlike other organic acids produced through a fermentation process, 3-hydroxypropionic acid exhibits high hydrophilicity, high solubility and reactivity in water. Accordingly, it is difficult to apply a conventional process of separating and purifying organic acids such as precipitation or extraction.
The reaction extraction method is a method of extracting an organic acid with an active diluent such as amine or alcohol having good reactivity with the organic acid, and thus is capable of selectively extracting the organic acid and shows relatively high extraction efficiency. Accordingly, an attempt has been made to apply the reaction extraction method to the separation and purification of 3HP, too. As one example, a method of using trioctylamine (TOA) as the amine has been proposed, but there are problems in that a 3HP extraction efficiency is low and a large amount of organic solvent is required. In addition, when tridecylamine is used as the amine, an extraction efficiency of 3HP is higher than that of TOA, but there is a problem of causing an emulsion phenomenon in which an organic phase and an aqueous phase are not separated during extraction.
Thus, there is a need to develop a process of recovering 3-hydroxypropionic acid with high purity and high yield from a raw material solution containing 3-hydroxypropionic acid, such as a microbial fermented solution, etc.

**SUMMARY OF THE INVENTION**

**TECHNICAL PROBLEM**

In the present disclosure, there are provided a process of recovering 3-hydroxypropionic acid including the step of producing a crystal of 3-hydroxypropionate, and the crystal of 3-hydroxypropionate.

**TECHNICAL SOLUTION**

In the present disclosure, there is provided a process of recovering 3-hydroxypropionic acid including the steps of: forming a crystal of 3-hydroxypropionate in a concentrate containing 3-hydroxypropionic acid in an amount of 300 g/L or more in the presence of an alkali metal salt; and separating the crystal of 3-hydroxypropionate from the concentrate

and converting it into 3-hydroxypropionic acid.

In the present disclosure, there is also provided a crystal of 3-hydroxypropionate of Structural formula 1 or Structural formula 2 below:

[Structural formula 1]     $Cation(3HP)_n$

[Structural formula 2]     $Cation(3HP)_n \cdot mH_2O$

wherein the Cation is a cation,

the 3HP is 3-hydroxypropionic acid bonding to the cation;
the n is a number of 3HPs bonding to the cation, which is an integer of 1 or more; and
the m is a number of water molecules in a hydrate, which is an integer of 1 or more.

Hereinafter, the process of recovering 3-hydroxypropionic acid and the crystal of 3-hydroxypropionate according to specific embodiments of the present invention will be described in more detail.

With regard to the steps constituting the preparation method described in this specification, it is not interpreted that one step and another step constituting one preparation method are limited to the order described in the specification, unless those steps are described to be sequential or consecutive or particularly mentioned otherwise. Accordingly, the order of the constituent steps of the preparation method can be changed within a range that can be easily understood by those skilled in the art, and in this case, accompanying changes apparent to those skilled in the art are included in the scope of the present disclosure.

According to one embodiment of the present disclosure, provided is a process of recovering 3-hydroxypropionic acid including the steps of: forming a crystal of 3-hydroxypropionate in a concentrate containing 3-hydroxypropionic acid in an amount of 300 g/L or more in the presence of an alkali metal salt; and separating the crystal of 3-hydroxypropionate from the concentrate and converting it into 3-hydroxypropionic acid.

The present inventors have confirmed through an experiment that crystals of 3-hydroxypropionate can be formed when 3-hydroxypropionic acid is enriched at a high concentration in the presence of an alkali metal salt, and have also confirmed through an experiment that the resulting crystals of 3-hydroxypropionate can be easily separated (and purified) from a liquid phase, and thus 3-hydroxypropionate can be recovered with high purity and high efficiency, thereby completing the present invention.

More specifically, a concentrate containing the 3-hydroxypropionic acid can include 3-hydroxypropionic acid at a concentration of 300 g/L or more, 350 g/L or more, 400 g/L or more, 450 g/L or more, or 500 g/L or more, and 900 g/L or less, 850 g/L or less, or 800 g/L or less.

It appears that the generation of the crystal of 3-hydroxypropionic acid is affected by the presence of an alkali metal salt, the concentration of 3-hydroxypropionic acid in the concentrate, and the like.

In addition, when the concentration of the crystal of 3-hydroxypropionic acid in the concentrate is higher than a water solubility of the crystal of 3-hydroxypropionic acid, the crystal of 3-hydroxypropionic acid can be more easily produced.

For example, the water solubility of $Ca(3HP)_2$, which is the crystal of 3-hydroxypropionic acid, is 450 g/L at room temperature, and thus when the concentration of 3-hydroxypropionic acid in the concentrate exceeds 450 g/L, the generation of $Ca(3HP)_2$ crystals can be promoted. In addition, the water solubility of $Mg(3HP)_2$, which is the crystal of 3-hydroxypropionic acid, is 250 g/L at room temperature, and thus when the concentration of 3-hydroxypropionic acid in the concentrate exceeds 250 g/L, the generation of $Mg(3HP)_2$ crystals can be promoted.

The crystal of 3-hydroxypropionate can be formed from the concentrate that satisfies the above-mentioned concentration while containing the alkali metal salt, and the alkali metal salt can be selected without limitation within the appropriate range for forming the crystal of 3-hydroxypropionate. For example, the alkali metal salt can include one or more cations selected from the group consisting of $Na^+$, $Mg^{2+}$ and $Ca^{2+}$, but when $Mg^{2+}$ or $Ca^{2+}$ is included, the crystal of 3-hydroxypropionate can be more effectively formed. For example, the alkali metal salt can be $Ca(OH)_2$, $Mg(OH)_2$ or a mixture thereof.

The alkali metal salt can be added and remain in the process of producing the fermented solution of 3-hydroxypropionic acid, or can be added during the process of forming a crystal of 3-hydroxypropionic acid in a concentrate containing the 3-hydroxypropionic acid in an amount of 300 g/L or more. In addition, the concentration of the alkali metal salt can be 10% to 100%, or 30% to 90% of the concentration of 3-hydroxypropionic acid and, for example, can be present in the concentrate at a concentration of 10 to 900 g/L, 50 to 800 g/L, 100 to 700 g/L, or 200 to 600 g/L.

The crystal of 3-hydroxypropionate can be in the form of Structural formula 1 or 2 below. In other words, the crystal of 3-hydroxypropionate can include 3-hydroxypropionate in the form of Structural formula 1 or 2 below.

In Structural formulas 1 and 2 below, Cation can represent a cation, the 3HP can represent 3-hydroxypropionic acid bonding to the cation, and n can represent a number of 3HPs bonding to the cation, which is an integer of 1 or more. In structural formula 2 below, m can represent a number of water molecules bonding to Cation(3HP)n in a hydrate, which is an integer of 1 or more. The cation can be, for example, $Na^+$, $Mg^{2+}$, or $Ca^{2+}$, but in the case of $Mg^{2+}$ or $Ca^{2+}$, the crystal of 3-hydroxypropionate can be more effectively formed.

[Structural formula 1]     $Cation(3HP)_n$

[Structural formula 2]     $Cation(3HP)_n \cdot mH_2O$

In addition, the step of forming of the crystal of 3-hydroxypropionate can further include the step of stirring the concentrate.

The stirring step can be performed at 0 to 70 °C, 0 to 60 °C, 0 to 50 °C, 0 to 40 °C, 0 to 35 °C, 0 to 30 °C, 10 to 70 °C, 10 to 60 °C, 10 to 50 °C, 10 to 40 °C, 10 to 35 °C, 10 to 30 °C, 15 to 70 °C, 15 to 60 °C, 15 to 50 °C, 15 to 40 °C, 15 to 35 °C, 15 to 30 °C, 20 to 70 °C, 20 to 60 °C, 20 to 50 °C, 20 to 40 °C, 20 to 35 °C, or 20 to 30 °C (e.g., at room temperature) and/or 100 to 2000 rpm, 100 to 1500 rpm, 100 to 1000 rpm, 100 to 500 rpm, 100 to 400 rpm, or 200 to 400 rpm (e.g., about 300 rpm).

The crystal of 3-hydroxypropionate can have a particle size distribution $D_{50}$ of 20 $\mu$m or more and 90 $\mu$m or less, 25 $\mu$m or more and 85 $\mu$m or less, 30 $\mu$m or more and 80 $\mu$m or less, or 35 $\mu$m or more and 75 $\mu$m or less.

In addition, the crystal of 3-hydroxypropionate can have a particle size distribution $D_{10}$ of 5 $\mu$m or more and 40 $\mu$m or less, 8 $\mu$m or more and 35 $\mu$m or less, or 10 $\mu$m or more and 30 $\mu$m or less, and the crystal of 3-hydroxypropionate can have a particle size distribution $D_{90}$ of 50 $\mu$m or more and 200 $\mu$m or less, 60 $\mu$m or more and 190 $\mu$m or less, 65 $\mu$m or more and 180 $\mu$m or less, or 70 $\mu$m or more and 175 $\mu$m or less.

The particle size distributions $D_{50}$, $D_{10}$, and $D_{90}$ can refer to particle diameters corresponding to 50%, 10%, and 90% of a volume accumulation in the particle size distribution curve of the particles, respectively, and the $D_{50}$, $D_{10}$, and $D_{90}$ can be measured, for example, by using a laser diffraction method. In general, the laser diffraction method can measure a particle diameter of several mm from a submicron region, and can obtain results of high reproducibility and high resolution.

If the crystal of 3-hydroxypropionate has excessively large particle size distributions $D_{50}$, $D_{10}$, and $D_{90}$, impurities to be removed during crystallization can be included in the crystal, resulting in poor purification efficiency. If the particle size distributions are excessively small, the crystal of 3-hydroxypropionate can have low permeability during filtration of the crystals.

Meanwhile, the crystal of 3-hydroxypropionate can have $(D_{90}-D_{10})/D_{50}$ of 1.00 or more and 3.00 or less, 1.20 or more and 2.80 or less, 1.40 or more and 2.60 or less, or 1.60 or more and 2.40 or less.

In addition, the crystal of 3-hydroxypropionate can have a mean diameter of volume distribution of 30 $\mu$m or more and 100 $\mu$m or less, 35 $\mu$m or more and 95 $\mu$m or less, or 40 $\mu$m or more and 90 $\mu$m or less, a mean diameter of number distribution of 1 $\mu$m or more and 30 $\mu$m or less, 3 $\mu$m or more and 25 $\mu$m or less, or 5 $\mu$m or more and 20 $\mu$m or less, and a mean diameter of area distribution of 10 $\mu$m or more and 70 $\mu$m or less, 15 $\mu$m or more and 60 $\mu$m or less, or 20 $\mu$m or more and 55 $\mu$m or less.

If the crystal of 3-hydroxypropionate has excessively large mean diameters of the volume distribution, number distribution, and area distribution, impurities to be removed during crystallization can be included in the crystal, resulting in poor purification efficiency. If the crystal have excessively small mean diameters of the volume distribution, number distribution, and area distribution, the crystal of 3-hydroxypropionate can have low permeability during filtration of the crystals.

In addition, the crystal of 3-hydroxypropionate can have an aspect ratio (LW Ratio; length to width ratio) and an average aspect ratio of 0.50 or more and 3.00 or less, 0.70 or more and 2.80 or less, or 1.00 or more and 2.50 or less, respectively, in the particle size distributions ($D_{10}$, $D_{50}$, $D_{90}$). If the crystal of 3-hydroxypropionate has an excessively large aspect ratio, a problem can occur to flowability and clogging during transport of the crystals, and if he crystals have an excessively small aspect ratio, permeability can be lowered during filtration of the crystals.

With regard to the crystal of 3-hydroxypropionate, a moisture content contained in the crystal can be measured by the Karl Fischer method, and the moisture content contained in the crystal of 3-hydroxypropionate can be 200 ppm or more and 5000 ppm or less, 250 ppm or more and 4800 ppm or less, 300 ppm or more and 4600 ppm or less, or 350 ppm or more and 4400 ppm or less.

In this case, the moisture contained in the crystal of 3-hydroxypropionate can refer to the attached moisture contained between the crystals rather than the crystal moisture (for example, $Ca(3HP)_2 \cdot 2H_2O$). In addition, if a moisture content contained in the crystal of 3-hydroxypropionate is too large, the crystal can be recovered in the form of slurry rather than a crystalline solid, or impurities can be contained in the moisture, which can cause a problem in that it reduces an improvement in purity.

In the process of recovering 3-hydroxypropionic acid according to above one embodiment, the crystal of 3-hydroxypropionate can include a radioactive isotope ($^{14}C$) as 3-hydroxypropionic acid is prepared through a process of fermenting a strain having a capacity to produce 3-hydroxypropionic acid, etc., as described below.

The radioactive isotope ($^{14}C$) can be nearly one per $10^{12}$ carbon atoms in the earth's atmosphere and have a half-life of about 5,700 years, and a carbon stock can be abundant in an upper atmosphere due to a nuclear reaction involving cosmic rays and common nitrogen ($^{14}N$). Meanwhile, in fossil fuels, radioactive isotopes have decayed long ago and thus a $^{14}C$ ratio can be substantially zero. When bio-derived raw materials are used as a raw material for 3-hydroxypropionic acid or when fossil fuels are used together, a content of radioactive carbon isotopes contained in 3-hydroxypropionic acid (percent modern carbon; pMC) and a content of biocarbon can be measured according to ASTM D6866-21 standard.

For example, carbon atoms included in the compound to be measured can be made into the form of graphite or carbon dioxide gas, and then measured with a mass spectrometer, or according to a liquid scintillation spectroscopy. In this case, two isotopes can be separated with an accelerator for separating $^{14}C$ ions from $^{12}C$ ions together with the mass spectrometer, etc., and thus the content and content ratio can be measured with a mass spectrometer.

The crystal of 3-hydroxypropionate can have a radioactive isotope content of 20 pMC (percent modern carbon) or more, 50 pMC or more, 90 pMC or more, or 100 pMC or more as measured by ASTM D6866-21 standard, and can have a biocarbon content of 20 wt% or more, 50 wt% or more, 80 wt% or more, 90 wt% or more, or 95 wt% or more.

The radioactive isotope ratio (pMC) can refer to a ratio of the radioactive isotope ($^{14}C$) contained in the crystal of 3-hydroxypropionate and the radioactive isotope ($^{14}C$) of the modern reference material, in which the effectiveness of a nuclear test program in the 1950s is continuing and not extinguished, and thus can be greater than 100%.

In addition, the biocarbon content can refer to the content of biocarbon with respect to a total carbon content included in the crystal of 3-hydroxypropionate, and a higher value can correspond to an eco-friendly compound.

Meanwhile, if the crystal of 3-hydroxypropionate has an excessively small content of radioactive isotopes (pMC) and biocarbons, eco-friendliness can be reduced and may not be regarded as a bio-derived material.

The crystal state of the crystal of 3-hydroxypropionate can be confirmed through a peak, etc., in an X-ray diffraction (XRD) graph.

For example, the crystal of 3-hydroxypropionate can show a peak between crystal lattices in a 2θ range of 8 to 22° in an X-ray diffraction (XRD) analysis.

For example, when the crystal of 3-hydroxypropionate to be formed is Mg(3HP)$_2$ due to magnesium hydroxide (Mg(OH)$_2$) contained in the concentrate, a peak between crystal lattices caused by a bond between 3-hydroxypropionic acid and magnesium can appear in the 2θ range of 8 to 15° in an X-ray diffraction (XRD) analysis for Mg(3HP)$_2$. This peak can indicate a result different from the result of X-ray diffraction (XRD) analysis for magnesium hydroxide (Mg(OH)$_2$) or magnesium sulfate (Mg(SO$_4$)). If a specific peak appears in the 2θ range of 8 to 15° as a result of X-ray diffraction (XRD) analysis, it can be confirmed that a crystal of Mg(3HP)$_2$ is formed.

Specifically, at least three, at least four or at least five peaks can appear in the 2θ range of 8 to 15° in an X-ray diffraction (XRD) analysis for Mg(3HP)$_2$. For example, each peak can appear in the 2θ range of 8.2 to 9.3°, 9.5 to 11.0°, 11.2 to 12.7°, 12.9 to 13.3°, or 13.5 to 14.8°.

In addition, when the crystal of 3-hydroxypropionate to be formed is Ca(3HP)$_2$ due to calcium hydroxide (Ca(OH)$_2$) contained in the concentrate, a peak between crystal lattices caused by a bond between 3-hydroxypropionic acid and calcium can appear in the 2θ range of 10 to 22° in an X-ray diffraction (XRD) analysis for Ca(3HP)$_2$. This peak can indicate a result different from the result of X-ray diffraction (XRD) analysis for calcium hydroxide (Ca(OH)$_2$) or calcium sulfate (Ca(SO$_4$)). If a specific peak appears in the 2θ range of 10 to 22° as a result of X-ray diffraction (XRD) analysis, it can be confirmed that a crystal of Ca(3HP)$_2$ is formed.

Specifically, at least three, at least five, at least seven, or at least nine peaks can appear in the 2θ range of 10.0 to 22° in an X-ray diffraction (XRD) analysis for Ca(3HP)$_2$. For example, each peak can appear in the 2θ range of 10.0 to 11.0°, 11.1 to 11.6°, 11.6 to 12.5°, 12.7 to 13.6°, 13.8 to 16.0°, 17.0 to 18.0°, 19.0 to 19.8°, 20.2 to 21.2°, or 21.5 to 22.0°.

Meanwhile, the incident angle (θ) can refer to an angle between a crystal plane and X-rays when X-rays are irradiated to a specific crystal plane. As a two-fold value (2θ) of the incident angle in X-rays, which is a horizontal axis (x axis), increases in a positive direction in a graph in which a horizontal axis (x axis) on an x-y plane is a two-fold value (2θ) of the incident angle in X-rays and a vertical axis (y axis) on an x-y plane is diffraction intensity, the peak can refer to a point where a first differential value of a two-fold (2θ) value of the incident angle in X-rays, which is a horizontal axis (x axis), for diffraction intensity, which is a vertical axis (y axis), is changed from a positive value to a negative value and the first differential value (slope of the tangent line, dy/dx) becomes zero.

In addition, the crystal of 3-hydroxypropionate can have an interatomic spacing (d value) of 1.00 Å or more and 15.00 Å or less, 1.50 Å or more and 13.00 Å or less, 2.00 Å or more and 11.00 Å or less, or 2.50 Å or more and 10.00 Å or less in a crystal derived from X-ray diffraction (XRD) analysis.

For example, when the crystal of 3-hydroxypropionate is Mg(3HP)$_2$, a peak shown in the 2θ range of 8 to 15° can have an interatomic spacing (d value) of 1.00 Å or more and 15.00 Å or less, 2.00 Å or more and 13.00 Å or less, 4.00 Å or more and 11.00 Å or less, or 5.50 Å or more and 10.00 Å or less in a crystal.

In addition, when the crystal of 3-hydroxypropionate is Ca(3HP)$_2$, a peak shown in the $2\theta$ range of 10 to 22° can have an interatomic spacing (d value) of 1.00 Å or more and 15.00 Å or less, 2.00 Å or more and 13.00 Å or less, 3.00 Å or more and 10.00 Å or less, 3.40 Å or more and 9.00 Å or less, or 4.00 Å or more and 8.50 Å or less in a crystal.

Furthermore, the crystal of 3-hydroxypropionate can have a glass transition temperature of -55 °C or more and -30 °C or less, a melting point of 30 °C or more and 170 °C or less, and a crystallization temperature of 25 °C or more and 170 °C or less.

With regard to the crystal of 3-hydroxypropionate, the glass transition temperature, melting point, and crystallization temperature can be measured by differential scanning calorimetry (DSC), and a temperature increasing rate can be 1 to 20 °C/min when measured. In addition, the crystal of 3-hydroxypropionate can have a glass transition temperature of -55 °C or more and -30 °C or less, -50 °C or more and -35 °C or less, or -45 °C or more and - 40 °C or less. Furthermore, the crystal of 3-hydroxypropionate can have a melting point of 30 °C or more and 170 °C or less, 31 °C or more and 160 °C or less, or 32 °C or more and 150 °C or less. Moreover, the crystal of 3-hydroxypropionate can have a crystallization temperature of 25 °C or more and 170 °C or less, 27 °C or more and 160 °C or less, or 30 °C or more and 150 °C or less. Besides, the crystal of 3-hydroxypropionate can have a crystallization stable region of -40 °C to 150 °C.

The process of recovering 3-hydroxypropionic acid according to the embodiment can include the steps of producing a fermented solution of 3-hydroxypropionic acid by fermenting a strain having a capacity to produce 3-hydroxypropionic acid; and forming a concentrate containing 3-hydroxypropionic acid in an amount of 300 g/L or more by concentrating the fermented solution before the step of producing the crystal of 3-hydroxypropionate.

The strain having a 3-hydroxypropionic acid-producing capacity can include a gene encoding at least one or two proteins selected from the group consisting of glycerol dehydratase and aldehyde dehydrogenase.

In one example, the 3-hydroxypropionic acid-producing strain can further include a gene (gdrAB) encoding a glycerol dehydratase reactivating enzyme (GdrAB). In one example, the 3-hydroxypropionic acid-producing strain can be a strain capable of additionally biosynthesizing vitamin B12.

The glycerol dehydratase can be encoded by a dhaB (GenBank accession no. U30903.1) gene, but is not limited thereto. The dhaB gene can be an enzyme derived from Klebsiella pneumonia, but is not limited thereto. The gene encoding the glycerol dehydratase can include a gene encoding dhaB1, dhaB2 and/or dhaB3. The glycerol dehydratase protein and the gene encoding the same can have a mutation of a gene and/or amino acid sequence within a range of maintaining an enzyme activity for decomposing glycerol into 3-hydroxypropanal (3-HPA) and water ($H_2O$).

The gene (aldH) encoding the aldehyde dehydrogenase (ALDH) can be, for example, aldH (GenBank Accession no. U00096.3; EaldH) gene derived from Escherichia coli or E. coli K12 MG1655 cell line, puuC gene derived from K. pneumoniae, and/or KGSADH gene derived from Azospirillum brasilense, but is not limited thereto. The aldehyde dehydrogenase protein and the gene encoding the same can have a mutation of a gene and/or amino acid sequence within a range of maintaining an activity for producing 3-hydroxypropionic acid from 3-hydroxypropanal.

A medium for producing the fermented solution can be selected without limitation in the appropriate range for producing 3-hydroxypropionic acid. In one example, the medium can include glycerol as a carbon source. In another example, the medium can be crude glycerol and/or pre-treated crude glycerol, but is not limited thereto. In one example, the medium for production can further include vitamin B12.

In the step of producing a 3-hydroxypropionic acid fermented solution by fermenting the strain having a capacity to produce 3-hydroxypropionic acid, the concentration of 3-hydroxypropionic acid contained in the 3-hydroxypropionic acid fermented solution can be 1 to 200 g/L, 10 to 150 g/L, 30 to 130 g/L, or 40 to 100 g/L.

In addition, the fermentation can be neutral fermentation, and the pH can be maintained in the range of 6 to 8, 6.5 to 8, 6 to 7.5, or 6.5 to 7.5 during fermentation, but is not limited thereto. The pH range can be appropriately adjusted as necessary. The alkali metal salt can be added for the neutral fermentation. The alkali metal salt can include $Mg^{2+}$, $Ca^2$, or a mixture thereof. In addition, the alkali metal salt can be Ca(OH)$_2$ or Mg(OH)$_2$, but is not limited thereto.

The process of recovering 3-hydroxypropionic acid according to the embodiment can further include the steps of: after producing the 3-hydroxypropionic acid fermented solution, removing (separating) cells from the fermented solution; purifying and/or decolorizing the fermented solution and/or the fermented solution without the cells; and filtering the fermented solution and/or the fermented solution without the cells.

The removal (separation) of the cells can be performed by selecting a method known in the art without limitation within the appropriate range of removing the cells (strains). In one example, the separation of the cells can be performed by centrifugation.

The purifying and/or decolorizing of the fermented solution and/or the fermented solution without the cells can be performed by selecting a method known in the art without limitation within the appropriate scope of purifying the fermented solution and, for example, can be performed by removing the activated carbon after mixing with the fermented solution, but is not limited thereto.

The filtering of the fermented solution and/or the fermented solution without the cells can be performed by selecting a method known in the art without limitation within the appropriate scope of removing solid impurities, removing proteins and/or materials with hydrophobic functional groups, and/or decoloration and, for example, can be performed by filter filtration, and/or activated carbon filtration method, but is not limited thereto.

The process of recovering 3-hydroxypropionic acid according to the embodiment can include the step of forming a concentrate containing 3-hydroxypropionic acid in an amount of 300 g/L or more by concentrating the fermented solution after producing a 3-hydroxypropionic acid fermented solution by fermenting the strain having a capacity to produce 3-hydroxypropionic acid.

Concentration of the fermented solution can be performed by evaporating the fermented solution (e.g., a liquid component of the fermented solution).

The concentration can be performed by any means commonly available to evaporate the liquid component of the fermented solution, for example, concentration by rotary evaporation, concentration by evaporation, vacuum concentration, concentration under reduced pressure, etc., but is not limited thereto.

In one example, the concentration of 3-hydroxypropionic acid in the fermented solution after concentration can be increased 2 to 50 times, 2 to 40 times, 2 to 30 times, 2 to 20 times, 2 to 10 times, 5 to 50 times, 5 to 40 times, 5 to 30 times, 5 to 20 times, or 5 to 10 times compared to before concentration.

As described above, after concentrating the fermented solution to form a concentrate containing 3-hydroxypropionic acid in an amount of 300 g/L or more, the crystal of 3-hydroxypropionate can be formed in a concentrate containing 3-hydroxypropionic acid in an amount of 300 g/L or more in the presence of an alkali metal salt.

In addition, after forming the crystal of 3-hydroxypropionate, it is possible to separate the crystal of 3-hydroxypropionate from the concentrate and convert it into 3-hydroxypropionic acid.

Separating the crystal of 3-hydroxypropionate from the concentrate can be performed by selecting a method known in the art to which the present disclosure pertains without limitation within the appropriate range of separating crystals. In one example, the recovery of the crystal of 3-hydroxypropionate can be performed by drying (e.g., drying by heating, etc.) and/or filtration, but is not limited thereto.

In addition, converting (purifying) the separated crystal of 3-hydroxypropionate into 3-hydroxypropionic acid can be performed by selecting a method known in the art without limitation within the appropriate scope of purifying 3-hydroxypropionic acid. For example, at least one of the methods listed below can be used, but is not limited thereto.

For example, there can be

i) a method of protonating 3-hydroxypropionic acid by removing cations through a cation exchange resin;
ii) a method of protonating 3-hydroxypropionic acid by extracting cations (e.g., Mg, Ca, etc.) with an organic solvent by using liquid cation exchange; and
iii) a method of protonating 3-hydroxypropionic acid by titration with an acid (e.g., sulfuric acid, etc.) to prepare a salt (e.g., $CaSO_4$(s) or $MgSO_4$(s)).

In the process of recovering 3-hydroxypropionic acid (3HP) according to the embodiment, the recovery rate of 3-hydroxypropionic acid can be calculated by the equation 1 below.

[Equation 1]

$$\text{3HP recovery rate (\%)} = \{(\text{3HP content in crystal})/(\text{3HP content in fermented solution before crystallization})\}*100$$

In one example, the recovery rate of 3-hydroxypropionic acid in the process of recovering 3-hydroxypropionic acid provided by the present disclosure can be 40% or more, 50% or more, 60% or more, 70% or more, 80% or more, or 90% or more, for example, 40 to 99.9%, 50 to 99.9%, 60 to 99.9%, 70 to 99.9%, 80 to 99.9%, 90 to 99.9%, 40 to 99%, 50 to 99%, 60 to 99%, 70 to 99%, 80 to 99%, 90 to 99%, 40 to 97%, 50 to 97%, 60 to 97%, 70 to 97%, 80 to 97%, 90 to 97%, 40 to 95%, 50 to 95%, 60 to 95%, 70 to 95%, 80 to 95%, or 90 to 95%, but is not limited thereto. The recovery rate can be calculated based on weight.

In addition, in the process of recovering 3-hydroxypropionic acid (3HP), the purity of 3-hydroxypropionate contained in the crystal of 3-hydroxypropionate can be calculated as a percentage (%) of the mass of a compound having the above structural formula 1 and/or 2 to the mass of the total recovered crystals. In one example, in the process of recovering 3-hydroxypropionic acid provided by the present disclosure, the purity of 3-hydroxypropionate contained in the crystal of 3-hydroxypropionate to be produced can be 70% or more, 80% or more, 90% or more, 70 to 99.9%, 80 to 99.9%, 90 to 99.9%, 70 to 99%, 80 to 99%, or 90 to 99%, but is not limited thereto.

According to another embodiment of the present disclosure, provided is a crystal of 3-hydroxypropionate of Structural formula 1 or 2 below.

[Structural formula 1]        Cation(3HP)$_n$

[Structural formula 2]        Cation(3HP)$_n \cdot m$H$_2$O

In Structural formulas 1 and 2, Cation can represent a cation, the 3HP can represent 3-hydroxypropionic acid bonding to the cation, and n can represent a number of 3HPs bonding to the cation, which is an integer of 1 or more. In Structural formula 2, m can represent a number of water molecules bonding to Cation(3HP) in a hydrate, which can be an integer of 1 or more. The cation can be, for example, Na$^+$, Mg$^{2+}$, or Ca$^{2+}$, but in the case of Mg$^{2+}$ or Ca$^{2+}$, the crystal of 3-hydroxypropionate can be more effectively formed.

The crystal of 3-hydroxypropionate can be formed in the process of recovering 3-hydroxypropionic acid according to the embodiment and, for example, can be formed in the forming of the crystal of 3-hydroxypropionate in the concentrate containing 3-hydroxypropionic acid in an amount of 300 g/L or more in the presence of the alkali metal salt.

The crystal of 3-hydroxypropionate can have a particle size distribution D$_{50}$ of 20 μm or more and 90 μm or less, 25 μm or more and 85 μm or less, 30 μm or more and 80 μm or less, or 35 μm or more and 75 μm or less, a particle size distribution D$_{10}$ of 5 μm or more and 40 μm or less, 8 μm or more and 35 μm or less, or 10 μm or more and 30 μm or less, and a particle size distribution D$_{90}$ of 50 μm or more and 200 μm or less, 60 μm or more and 190 μm or less, 65 μm or more and 180 μm or less, or 70 μm or more and 175 μm or less.

In addition, the crystal of 3-hydroxypropionate can have (D$_{90}$-D$_{10}$)/D$_{50}$ of 1.00 or more and 3.00 or less, 1.20 or more and 2.80 or less, 1.40 or more and 2.60 or less, or 1.60 or more and 2.40 or less.

Furthermore, the crystal of 3-hydroxypropionate can have a mean diameter of volume distribution of 30 μm or more and 100 μm or less, 35 μm or more and 95 μm or less, or 40 μm or more and 90 μm or less, a mean diameter of number distribution of 1 μm or more and 30 μm or less, 3 μm or more and 25 μm or less, or 5 μm or more and 20 μm or less, and a mean diameter of area distribution of 10 μm or more and 70 μm or less, 15 μm or more and 60 μm or less, or 20 μm or more and 55 μm or less.

In addition, the crystal of 3-hydroxypropionate can have an aspect ratio (LW Ratio; length to width ratio) and an average aspect ratio of 0.50 or more and 3.00 or less, 0.70 or more and 2.80 or less, or 1.00 or more and 2.50 or less, respectively, in the particle size distributions (D$_{10}$, D$_{50}$, D$_{90}$).

A moisture content contained in the crystal of 3-hydroxypropionate can be 200 ppm or more and 5000 ppm or less, 250 ppm or more and 4800 ppm or less, 300 ppm or more and 4600 ppm or less, or 350 ppm or more and 4400 ppm or less.

The crystal of 3-hydroxypropionate can have a radioactive isotope content of 20 pMC (percent modern carbon) or more, 50 pMC or more, 90 pMC or more, or 100 pMC or more as measured by ASTM D6866-21 standard, and can have a biocarbon content of 20 wt% or more, 50 wt% or more, 80 wt% or more, 90 wt% or more, or 95 wt% or more.

In addition, the crystal of 3-hydroxypropionate can have a peak between crystal lattices in the 2θ range of 8 to 22° in an X-ray diffraction (XRD) analysis and can have an interatomic spacing (d value) of 1.00 Å or more and 15.00 Å or less, 1.50 Å or more and 13.00 Å or less, 2.00 Å or more and 11.00 Å or less, or 2.50 Å or more and 10.00 Å or less in a crystal derived from XRD analysis.

Furthermore, the crystal of 3-hydroxypropionate can have a glass transition temperature of -55 °C or more and -30 °C or less, a melting point of 30 °C or more and 170 °C or less, and a crystallization temperature of 25 °C or more and 170 °C or less.

Moreover, the 3-hydroxypropionate included in the crystal of 3-hydroxypropionate can have a purity of 70% or more, 80% or more, 90% or more, 70 to 99.9%, 80 to 99.9%, 90 to 99.9%, 70 to 99%, 80 to 99%, or 90 to 99%.

In addition, with regard to the above-mentioned crystal of 3-hydroxypropionate, conditions for measuring the particle size distributions D$_{10}$, D$_{50}$, D$_{90}$, (D$_{90}$-D$_{10}$)/D$_{50}$, mean diameters of the volume distribution, number distribution and area distribution, aspect ratio and average aspect ratio in the particle size distribution (D$_{10}$, D$_{50}$, D$_{90}$), moisture content, radioactive isotope content, biocarbon content, XRD analysis results (interatomic spacing in crystal (d value), peak shown in a specific 2θ range), glass transition temperature, melting point, crystallization temperature, purity, and the like can be the same as those described above in the process of recovering 3-hydroxypropionic acid according to the embodiment.

According to another embodiment of the present disclosure, provided is the crystal of 3-hydroxypropionate which includes an alkali metal salt of 3-hydroxypropionic acid or a hydrate thereof, and has a biocarbon content of 20 wt% or more as measured by ASTM D6866-21 standard.

The alkali metal salt of 3-hydroxypropionic acid can be a calcium salt or magnesium salt of 3-hydroxypropionic acid.

In addition, the alkali metal salt of 3-hydroxypropionic acid can be of Structural formula 1 below, and the hydrate of alkali metal salt of 3-hydroxypropionic acid can be of Structural formula 2 below. The following Cation can be Mg$^{2+}$ or Ca$^{2+}$.

[Structural formula 1]        Cation(3HP)$_n$

[Structural formula 2]        Cation(3HP)$_n$·mH$_2$O

In addition, the crystal of 3-hydroxypropionate can have a radioactive isotope content of 20 pMC (percent modern carbon) or more, 50 pMC or more, 90 pMC or more, or 100 pMC or more as measured by ASTM D6866-21 standard, and can have a biocarbon content of 20 wt% or more, 50 wt% or more, 80 wt% or more, 90 wt% or more, or 95 wt% or more.

Furthermore, the crystal of 3-hydroxypropionate can have a mean diameter of volume distribution of 30 μm or more and 100 μm or less, 35 μm or more and 95 μm or less, or 40 μm or more and 90 μm or less, a mean diameter of number distribution of 1 μm or more and 30 μm or less, 3 μm or more and 25 μm or less, or 5 μm or more and 20 μm or less, and a mean diameter of area distribution of 10 μm or more and 70 μm or less, 15 μm or more and 60 μm or less, or 20 μm or more and 55 μm or less.

Moreover, the crystal of 3-hydroxypropionate can have an aspect ratio (LW Ratio; length to width ratio) and an average aspect ratio of 0.50 or more and 3.00 or less, 0.70 or more and 2.80 or less, or 1.00 or more and 2.50 or less, respectively, in the particle size distributions (D10, D50, D90).

The crystal of 3-hydroxypropionate can have a particle size distribution D$_{50}$ of 20 μm or more and 90 μm or less, 25 μm or more and 85 μm or less, 30 μm or more and 80 μm or less, or 35 μm or more and 75 μm or less, a particle size distribution D$_{10}$ of 5 μm or more and 40 μm or less, 8 μm or more and 35 μm or less, or 10 μm or more and 30 μm or less, and a particle size distribution D$_{90}$ of 50 μm or more and 200 μm or less, 60 μm or more and 190 μm or less, 65 μm or more and 180 μm or less, or 70 μm or more and 175 μm or less.

In addition, the crystal of 3-hydroxypropionate can have (D$_{90}$-D$_{10}$)/D$_{50}$ of 1.00 or more and 3.00 or less, 1.20 or more and 2.80 or less, 1.40 or more and 2.60 or less, or 1.60 or more and 2.40 or less.

A moisture content contained in the crystal of 3-hydroxypropionate can be 200 ppm or more and 5000 ppm or less, 250 ppm or more and 4800 ppm or less, 300 ppm or more and 4600 ppm or less, or 350 ppm or more and 4400 ppm or less.

In addition, the crystal of 3-hydroxypropionate can have a peak between crystal lattices in the 2θ range of 8 to 22° in an X-ray diffraction (XRD) analysis and can have an interatomic spacing (d value) of 1.00 Å or more and 15.00 Å or less, 1.50 Å or more and 13.00 Å or less, 2.00 Å or more and 11.00 Å or less, or 2.50 Å or more and 10.00 Å or less in a crystal derived from XRD analysis.

Furthermore, the crystal of 3-hydroxypropionate can have a glass transition temperature of -55 °C or more and -30 °C or less, a melting point of 30 °C or more and 170 °C or less, and a crystallization temperature of 25 °C or more and 170 °C or less.

Moreover, the 3-hydroxypropionate included in the crystal of 3-hydroxypropionate can have a purity of 70% or more, 80% or more, 90% or more, 70 to 99.9%, 80 to 99.9%, 90 to 99.9%, 70 to 99%, 80 to 99%, or 90 to 99%.

With regard to the above-mentioned crystal of 3-hydroxypropionate, conditions for measuring the particle size distributions D$_{10}$, D$_{50}$, D$_{90}$, (D$_{90}$-D$_{10}$)/D$_{50}$, mean diameters of the volume distribution, number distribution and area distribution, aspect ratio and average aspect ratio in the particle size distribution (D$_{10}$, D$_{50}$, D$_{90}$), moisture content, radioactive isotope content, biocarbon content, XRD analysis results (interatomic spacing in crystal (d value), peak shown in a specific 2θ range), glass transition temperature, melting point, crystallization temperature, purity, and the like as well as detailed description thereof can be the same as those described above in the process of recovering 3-hydroxypropionic acid according to the embodiment.

**ADVANTAGEOUS EFFECTS**

The crystal of 3-hydroxypropionate provided by the present disclosure can include 3-hydroxypropionate with high purity and have a specific particle size or shape, thereby facilitating a filtration from impurities such as fermentation by-products, and/or additives. In addition, the process of recovering (separating, purifying) 3-hydroxypropionic acid can easily separate fermentation by-products and/or additives through the crystallization of 3-hydroxypropionate, and thus 3-hydroxypropionate can be recovered with high purity and the process can be performed without an organic solvent to simplify the process of separating and purifying 3-hydroxypropionic acid, thereby reducing the cost of purifying 3-hydroxypropionic acid.

**BRIEF DESCRIPTION OF THE DRAWINGS**

FIGS. 1 and 2 are graphs of showing results of an X-ray diffraction (XRD) analysis on the crystal of 3-hydroxypropionate ($Ca(3HP)_2$) of Example 1.
FIGS. 3 and 4 are graphs of showing results of an X-ray diffraction (XRD) analysis on the crystal of 3-hydroxypropionate ($Mg(3HP)_2$) of Example 2.
FIG. 5 is a graph of showing results of a differential scanning calorimetry (DSC) analysis on the crystal of 3-hydroxypropionate ($Ca(3HP)_2$) of Example 1.
FIG. 6 is a graph of showing results of a differential scanning calorimetry (DSC) analysis on the crystal of 3-hydroxypropionate ($Mg(3HP)_2$) of Example 2.

**DETAILED DESCRIPTION**

Hereinafter, the present disclosure will be described in more detail with reference to examples. However, the following examples are provided only for the purpose of illustrating the present disclosure, and thus the scope of the present disclosure is not limited thereto.

In this disclosure, all temperatures are described in degrees Celsius unless otherwise specified.

**Preparation Example 1. Preparation of strains for production of 3-hydroxypropionic acid**

Prepared was a recombinant vector incorporating a gene encoding glycerol dehydratase and aldehyde dehydrogenase, which are known to produce 3-hydroxypropionic acid (3HP) using glycerol as a substrate. The prepared recombinant vector was introduced into the *E. coli* W3110 strain to prepare a 3-hydroxypropionic acid-producing strain.

More specifically, pCDF_J23101_dhaB_gdrAB_J23100_aldH_btuR vector, obtained by cloning BtuR gene encoding adenosyltransferase into plasmid pCDF including a gene (dhaB) encoding glycerol dehydratase, a gene (aldH) encoding aldehyde dehydrogenase and a gene (gdrAB) encoding glycerol dehydratase reactivase, was introduced into W3110 strain (KCCM 40219) by electroporation using an electroporation device (Bio-Rad, Gene Pulser Xcell) to prepare a 3-hydroxypropionic acid-producing strain. The process of preparing the 3-hydroxypropionic acid-producing strain of this Preparation Example 1, and the vectors, primers and enzymes used were performed with reference to Example 1 of Korean Patent Publication No. 10-2020-0051375 (incorporated herein by reference).

**Example 1. Preparation of $Ca(3HP)_2$ crystals**

The strain for producing 3-hydroxypropionic acid prepared in Preparation Example 1 was fermented and cultured at 35 °C in a 5 L fermenter by using unrefined glycerol as a carbon source, so as to produce 3-hydroxypropionic acid. In order to prevent a decrease in pH due to the production of 3-hydroxypropionic acid, calcium hydroxide ($Ca(OH)_2$), an alkali metal salt, was added to maintain a neutral pH during fermentation.

After fermentation culture, cells were removed by centrifugation (4000 rpm, 10 minutes, 4 °C), and primary fermented solution purification (primary purification) was performed by using activated carbon. Specifically, activated carbon was added to the fermented solution, from which the cells were removed by centrifugation, mixed well, and then centrifuged again to separate the activated carbon. After that, the fermented solution, from which the activated carbon was separated, was filtered through a 0.7 $\mu$m filter paper with a vacuum pump to purify the 3-hydroxypropionic acid fermented solution.

The concentration of 3-hydroxypropionic acid in the fermented solution obtained after the primary purification was at a level of 50 to 100 g/L, and the fermented solution was concentrated at a concentration of 600 g/L using a rotary evaporator (50 °C, 50 mbar) to prepare a concentrate, which was then stirred at room temperature (300 rpm) to produce $Ca(3HP)_2$ crystals. At this time, the concentration of the alkali metal salt in the concentrate was 493.3 g/L (based on $Ca(OH)_2$).

The resulting crystals were washed three times with ethanol (EtOH) and dried in an oven at 50 °C to finally recover crystals. The weight of the crystals was measured, the crystals were dissolved in water, and the amount of 3-hydroxypropionic acid (3HP) was quantified with high performance liquid chromatography (HPLC) to measure the amount of 3-hydroxypropionic acid in the crystals, after which a recovery rate was measured (Equation 1) compared to the amount of 3-hydroxypropionic acid measured by HPLC in the fermented solution.

[Equation 1]

3HP recovery rate (%) = {(3HP content in crystal)/(3HP content in fermented solution before crystallization)}*100

The recovery rate of 3-hydroxypropionic acid recovered as crystals was measured to be about 92%.

**Example 2. Preparation of Mg(3HP)$_2$ crystals**

A fermentation process was performed in substantially the same manner as in above Example 1, except for using Mg(OH)$_2$ instead of Ca(OH)$_2$ as an alkali metal salt, followed by concentration to produce and recover Mg(3HP)$_2$ crystals.
Table 1 below shows the recovery rates of Mg(3HP)$_2$ crystals for each concentration of each concentrate.

Table 1

| Concentration (g/L) | 3HP recovery rate through crystals (%) |
|---|---|
| 461.2 | 92.4 |
| 527.9 | 94.3 |
| 576.2 | 95.9 |
| 800.0 | 96.5 |

As can be seen in Table 1 above, at least 90% of 3-hydroxypropionic acid was recovered as a result of crystallization after concentration and thus it was confirmed that the recovery rate of 3-hydroxypropionic acid through crystallization was excellent.

**Comparative Example 1. Titration with sulfuric acid after fermentation**

A fermentation process was performed in the same manner as in above Example 1, and a fermented solution was titrated to pH 2 with sulfuric acid (H$_2$SO$_4$). The gypsum (Ca(SO)$_4$) generated in the titration process was removed to proceed with protonation of 3-hydroxypropionic acid.
The fermented solution, from which the gypsum had been removed, was first purified and concentrated in substantially the same manner as in above Example 1, so as to confirm whether crystals were formed or not.
However, in the case of concentration at 600 g/L (based on 3HP) during the concentration process, no crystals were formed, and even when concentration was performed up to 1000 g/L (based on 3HP), formation of crystals was not confirmed.

**<Test Example>**

**1. Analysis of purity of 3-hydroxypropionate in crystals**

Ion chromatography (IC) analysis (Dionex Aquion; Eluent: 20 mM Methanesulfonic Acid; Column: Dionex IonPac CS12A; Flow Rate: 1.0 ml/min; Suppressor: Dionex CERS 500 4mm; Current: 59 mA) was performed for a cation analysis of the 3-hydroxypropionate crystals obtained in above Examples 1 and 2, so as to confirm the purity of each 3-hydroxypropionate in the crystals.
Table 2 below shows the results of cation analysis of Ca(3HP)$_2$ crystals of Example 1, and table 3 below shows the results of measuring purity of 3-hydroxypropionate for each concentration of the Mg(3HP)$_2$ crystals of Example 2.

Table 2

| Cation type | Ca(3HP)$_2$ crystals | |
|---|---|---|
| | ppm | Composition (%) |
| Na$^+$ | 3.06 | 0.81 |
| Mg$^{2+}$ | 1.26 | 0.33 |
| Ca$^{2+}$ | 374.42 | 98.53 |
| K$^+$ | 1.26 | 0.33 |
| Total Cation | 380.00 | 100.00 |

Table 3

| Concentration (g/L) of Example 2 | Purity of Mg(3HP)$_2$ |
|---|---|
| 461.2 | 93.2 |
| 527.9 | 92.9 |
| 576.2 | 93.0 |
| 800.0 | 93.5 |

Referring to above Tables 2 and 3, it was confirmed that the purity of 3-hydroxypropionate in the crystals in Examples 1 and 2 was 92.9% or more. The purity of Ca(3HP)$_2$ was confirmed by a proportion of Ca$^{2+}$ among Cation types in above table 2, and as a result, it was confirmed that the purity was 98% or more, which is very excellent.

**2. Analysis of 3-hydroxypropionic acid in crystals**

High performance liquid chromatography (HPLC) analysis was performed to analyze the content of 3-hydroxypropionic acid in the crystal of 3-hydroxypropionate obtained in Examples 1 and 2. Specific conditions for HPLC performance are shown in table 4 below.

Table 4

| Category | Quality |
|---|---|
| Detector | RI / UV detector |
| Column | Bio-Rad Aminex HPX-87H Ion Exclusion Column 300 mm×7.8mm |
| Mobile Phase | 0.5 mM H$_2$SO$_4$ |
| Flow Rate | 0.4 mL/min |
| Run Time | 35 min |
| Column temperature | 35°C |
| Detector temperature | 35°C |
| Injection Volume | 10 $\mu\ell$ |

Table 5 below shows the results of HPLC performance for the crystals of Examples 1 and 2. In Table 5 below, 3-hydroxypropionate in the crystals can refer to Ca(3HP)$_2$ in Example 1 and Mg(3HP)$_2$ in Example 2.

Table 5

| Type | Crystal mass (g) | 3HP mass in crystal (g) | 3HP salt mass in crystal (g) | Crystal purity (%) |
|---|---|---|---|---|
| Example 1 | 0.2426 | 0.1880 | 0.2302 | 94.89 |
| Example 2 | 0.2580 | 0.2118 | 0.2404 | 93.20 |

Referring to Table 5 above, it was confirmed that the purity of 3HP salt in the crystals shown as a result of performing HPLC on the crystals of Examples 1 and 2 was 93.20% or more.

### 3. Analysis of particle size of 3-hydroxypropionate crystals

With regard to crystals of 3-hydroxypropionate $(Ca(3HP)_2)$ of Example 1 and crystals of 3-hydroxypropionate $(Mg(3HP)_2)$ of Example 2, $D_{10}$, $D_{50}$, $D_{90}$, mean diameters of the volume distribution, number distribution and area distribution were measured respectively with a size and shape particle analyzer (Microtrac TurboSync), and the results are shown in Table 6 below.

In addition, with regard to crystals of 3-hydroxypropionate of Examples 1 and 2, an aspect ratio (LW Ratio; length to width ratio) and an average aspect ratio at each of $D_{10}$, $D_{50}$ and $D_{90}$ were measured with the size and shape particle analyzer, and the results are shown in Table 7 below.

Table 6

|  | Concentration (g/L) | $D_{10}$ ($\mu$m) | $D_{50}$ ($\mu$m) | $D_{90}$ ($\mu$m) | Mean diameter of volume distribution (MV, $\mu$m) | Mean diameter of number distribution (MN, $\mu$m) | Mean diameter of area distribution (MA, $\mu$m) |
|---|---|---|---|---|---|---|---|
| Example 1 | 600 | 27.41 | 71.40 | 170.40 | 88.71 | 18.99 | 53.45 |
| Example 2 | 800 | 13.68 | 36.50 | 74.02 | 41.30 | 5.13 | 24.81 |

Table 7

|  | $D_{10}$ aspect ratio | $D_{50}$ aspect ratio | $D_{90}$ aspect ratio | Average aspect ratio |
|---|---|---|---|---|
| Example 1 | 27.41 | 71.40 | 170.40 | 88.71 |
| Example 2 | 13.68 | 36.50 | 74.02 | 41.30 |
| - Aspect ratio (LW Ratio; Length to width ratio) | | | | |

Referring to above Tables 6 and 7, in Examples 1 and 2, it was confirmed that the particle size distribution $D_{10}$ was 13.68 to 27.41 $\mu$m; the particle size distribution $D_{50}$ was 36.50 to 71.40 $\mu$m; the particle size distribution $D_{90}$ was 74.02 to 170.40; $(D_{90}-D_{10})/D_{50}$ was 2.00 for Example 1 and was 1.65 for Example 2; the mean diameter of the volume distribution was 41.30 to 88.71 $\mu$m; the mean diameter of the number distribution was 5.13 to 18.99 $\mu$m; the mean diameter of the area distribution was 24.81 to 53.45 $\mu$m; the $D_{10}$ aspect ratio (LW Ratio; length to width ratio) was 13.68 to 27.41; the $D_{50}$ aspect ratio was 36.50 to 71.40; the $D_{90}$ aspect ratio was 74.02 to 170.40; and the average aspect ratio was 41.30 to 88.71.

### 4. Analysis of moisture content of 3-hydroxypropionate crystals

The moisture content in 3-hydroxypropionate crystals $(Ca(3HP)_2)$ of Example 1 and in 3-hydroxypropionate crystals $(Mg(3HP)_2)$ of Example 2 was analyzed with Karl-Fischer titrator from Metrohm and is shown in Table 8 below.

Table 8

|  | Concentration (g/L) | Moisture content (ppm) |
|---|---|---|
| Example 1 | 600 | 351 |
| Example 2 | 800 | 4370 |

Referring to Table 8 above, it was confirmed that the moisture content of Examples 1 and 2 was 351 to 4370 ppm.

**5. Analysis of biocarbon content in 3-hydroxypropionate crystals**

A radioactive isotope ratio (pMC) and a biocarbon content in 3-hydroxypropionate crystals (Ca(3HP)$_2$) of Example 1 and in 3-hydroxypropionate crystals (Mg(3HP)$_2$) of Example 2 were analyzed with ASTM D 6866-21(Method B) and the results are shown in Table 9 below.

The biocarbon content can refer to the content of biocarbon contained in the 3-hydroxypropionate crystals of Examples 1 and 2, respectively, and the radioactive isotope ratio (pMC) can refer to the ratio of the radioactive isotope ($^{14}$C) contained in the 3-hydroxypropionate crystals and the radioactive isotope ($^{14}$C) of the modern reference material.

Table 9

| | Concentration (g/L) | Radioactive isotope ratio (pMC) | Biocarbon content (%) |
|---|---|---|---|
| Example 1 | 600 | 101.52 | 100 |
| Example 2 | 800 | 102.23 | 100 |
| - pMC: percent Modern Carbon | | | |

Referring to Table 9 above, it was confirmed that all crystals of 3HP salt of above Examples 1 and 2 had 101 pMC or more, and a biocarbon content of 100%.

**6. X-ray diffraction (XRD) analysis of 3-hydroxypropionate crystals**

The crystals prepared in Examples 1 and 2 were irradiated with Cu-K$\alpha$ rays having a wavelength of 1.54 Å to measure an X-ray diffraction (XRD) pattern in a reflection mode.

The measurement equipment used was a Bruker AXS D4 Endeavor XRD. The voltage and current used are 40 kV and 40 mA, respectively, and the optics and detector used are as follows:

- Primary (incident beam) optics: motorized divergence slit, soller slit 2.3°
- Secondary (diffracted beam) optics: soller slit 2.3°
- LynxEye detector (1D detector)

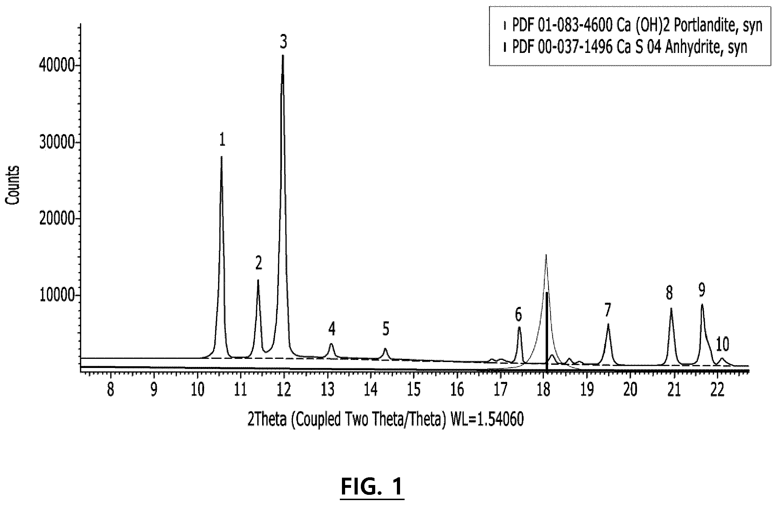

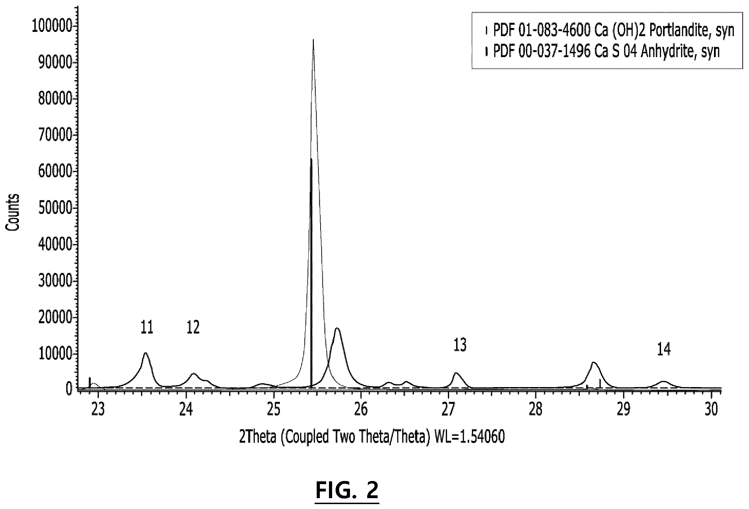

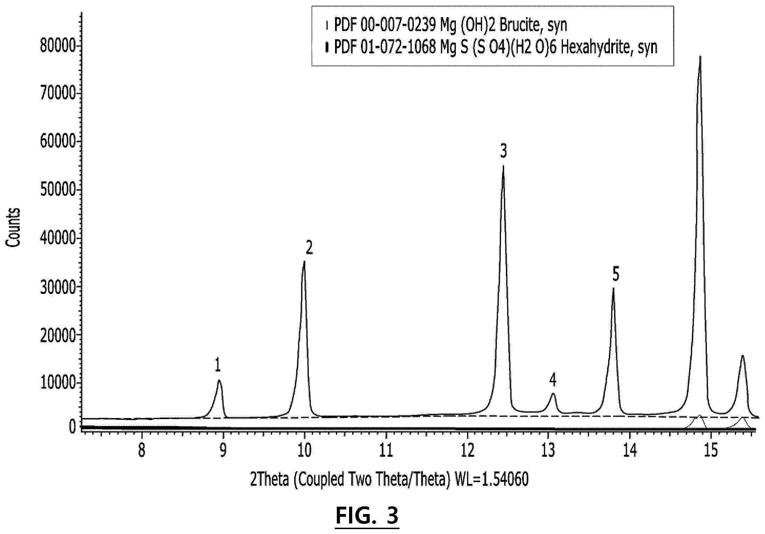

FIGS. 1 and 2 are graphs of showing results of an X-ray diffraction (XRD) analysis on the crystal of 3-hydroxypropionate of Example 1, and FIGS. 2 and 3 are graphs of showing results of an X-ray diffraction (XRD) analysis on the crystal of 3-hydroxypropionate of Example 2.

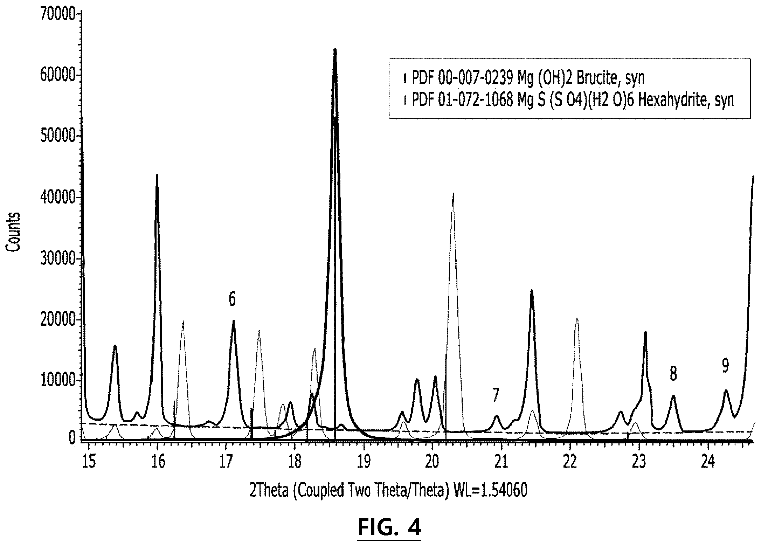

The 2θ value and lattice plane spacing (d values) of the numbered peaks in the XRD graphs of FIGS. 1 and 2 are shown in Table 10, and the 2θ value and lattice plane spacing of the numbered peaks in the XRD graphs of FIGS. 3 and 4 are shown in Table 11 below.

Table 10

| Peak No. | 2θ value (°) | Lattice plane spacing (d value, Å) | Peak No. | 2θ value (°) | Lattice plane spacing (d value, Å) |
|---|---|---|---|---|---|
| 1 | 10.0-11.0 | 8.37 | 8 | 20.6-21.2 | 4.24 |
| 2 | 11.1-11.6 | 7.75 | 9 | 21.5-22.0 | 4.10 |
| 3 | 11.6-12.4 | 7.38 | 10 | 22.0-22.4 | 4.01 |
| 4 | 12.9-13.3 | 6.75 | 11 | 23.2-23.7 | 3.78 |
| 5 | 14.1-14.5 | 6.17 | 12 | 23.9-24.3 | 3.69 |
| 6 | 17.2-17.6 | 5.08 | 13 | 26.9-27.2 | 3.29 |
| 7 | 19.2-19.8 | 4.55 | 14 | 29.2-29.6 | 3.03 |

Table 11

| Peak No. | 2θ value (°) | Lattice plane spacing (d value, Å) | Peak No. | 2θ value (°) | Lattice plane spacing (d value, Å) |
|---|---|---|---|---|---|
| 1 | 8.5-9.2 | 9.84 | 6 | 16.8-17.3 | 5.29 |

(continued)

| Peak No. | 2θ value (°) | Lattice plane spacing (d value, Å) | Peak No. | 2θ value (°) | Lattice plane spacing (d value, Å) |
|---|---|---|---|---|---|
| 2 | 9.5-10.3 | 8.82 | 7 | 20.7-21.0 | 4.24 |
| 3 | 12.0-12.7 | 7.10 | 8 | 23.3-23.7 | 3.85 |
| 4 | 12.9-13.2 | 6.77 | 9 | 24.0-24.4 | 3.66 |
| 5 | 13.5-14.0 | 5.95 | - | - | - |

Referring to Tables 10 and 11, it was confirmed that the crystal of 3-hydroxypropionate prepared in Example 1 was Ca(3HP)$_2$ and the crystal of 3-hydroxypropionate prepared in Example 2 was Mg(3HP)$_2$.

**7. Differential scanning calorimetry (DSC) analysis of 3-hydroxypropionate crystals**

With regard to the crystals of Examples 1 and 2, a melting point (Tm) of each crystal was confirmed with a differential scanning calorimeter (DSC (Q2000) manufactured by TA Instruments Co., Ltd.). In the case of Example 1, a glass transition temperature, a crystallization temperature, etc. were measured in the range of -60 °C to 150 °C at a temperature increasing rate of 10 °C/min. In the case of Example 2, a glass transition temperature, a crystallization temperature, etc. were measured in the range of -70 °C to 30 °C at a temperature increasing rate of 10 °C/min, and the results are shown in Table 12 below.

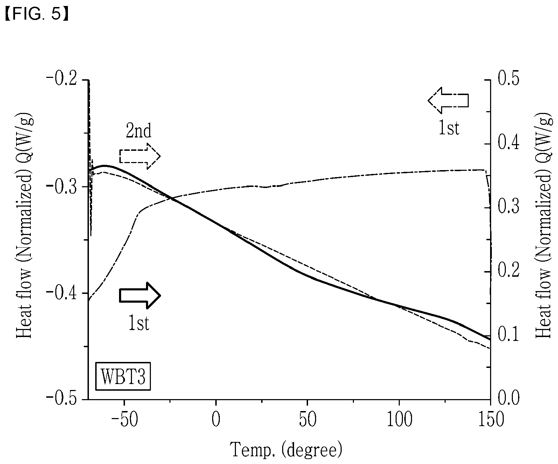

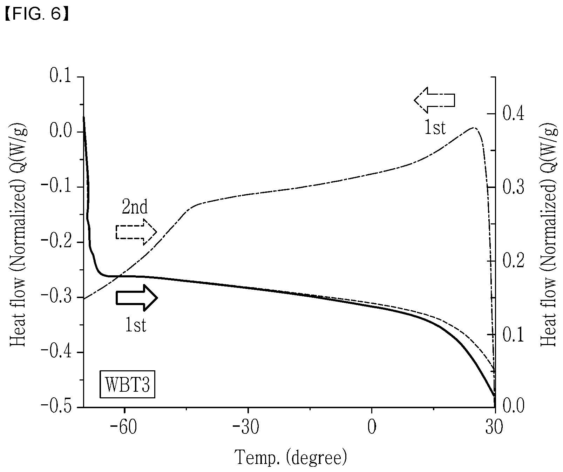

FIG. 5 is a graph of showing results of a differential scanning calorimeter (DSC) analysis on the crystal of 3-hydroxypropionate (Ca(3HP)$_2$) of Example 1, and FIG. 6 is a graph of showing results of a differential scanning calorimeter (DSC) analysis on the crystal of 3-hydroxypropionate (Mg(3HP)$_2$) of Example 2.

Table 12

| | Glass transition temperature (Tg, °C) | Melting Point (Tm, °C) | Crystallization temperature (Tc, °C) | Crystallization stable region | Metal |
|---|---|---|---|---|---|
| Example 1 | -45 | 32 | 30 | -40-30 | Ca |
| Example 2 | -40 | 155 | 150 | -20-150 | Mg |

Referring to Table 12, it was confirmed that the glass transition temperature of Examples 1 and 2 was -45 to -40 °C, the melting point was 32 to 155 °C, and the crystallization temperature was 30 to 150 °C.

**Claims**

1.  A process of recovering 3-hydroxypropionic acid, comprising the steps of:

    forming a crystal of 3-hydroxypropionate in a concentrate containing 3-hydroxypropionic acid in an amount of 300 g/L or more in the presence of an alkali metal salt; and
    separating the crystal of 3-hydroxypropionate from the concentrate; and
    converting it into 3-hydroxypropionic acid.

2.  The process of recovering 3-hydroxypropionic acid of claim 1,
    wherein the alkali metal salt comprises at least one cation selected from the group consisting of Na$^+$, Mg$^{2+}$ and Ca$^{2+}$.

3.  The process of recovering 3-hydroxypropionic acid of claim 1,
    wherein the alkali metal salt is Ca(OH)$_2$, Mg(OH)$_2$ or a mixture thereof.

4.  The process of recovering 3-hydroxypropionic acid of claim 1,
    wherein the concentrate comprises the 3-hydroxypropionic acid in an amount of 350 g/L or more and 900 g/L or less.

5.  The process of recovering 3-hydroxypropionic acid of claim 1,

wherein the crystal of 3-hydroxypropionate is in a form of structural formula 1 or structural formula 2:

[Structural formula 1]     Cation(3HP)$_n$

[Structural formula 2]     Cation(3HP)$_n$·mH$_2$O

wherein the Cation is a cation;

the 3HP is 3-hydroxypropionic acid bonding to the cation;
the n is a number of 3HPs bonding to the cation, which is an integer of 1 or more; and
the m is a number of water molecules in a hydrate, which is an integer of 1 or more.

6.  The process of recovering 3-hydroxypropionic acid of claim 1,
    wherein the crystal of 3-hydroxypropionate has a particle size distribution D$_{50}$ of 20 $\mu$m or more and 90 $\mu$m or less, and (D$_{90}$-D$_{10}$)/D$_{50}$ of 1.00 or more and 3.00 or less.

7.  The process of recovering 3-hydroxypropionic acid of claim 1,
    wherein the crystal of 3-hydroxypropionate has a mean diameter of volume distribution of 30 $\mu$m or more and 100 $\mu$m or less, a mean diameter of number distribution of 1 $\mu$m or more and 30 $\mu$m or less, and a mean diameter of area distribution of 10 $\mu$m or more and 70 $\mu$m or less.

8.  The process of recovering 3-hydroxypropionic acid of claim 1,
    wherein the crystal of 3-hydroxypropionate has an average aspect ratio (LW ratio; length to width ratio) of 0.50 or more and 3.00 or less.

9.  The process of recovering 3-hydroxypropionic acid of claim 1,
    wherein the crystal of 3-hydroxypropionate has a moisture content of 200 ppm or more and 5000 ppm or less as measured by a Karl Fischer method.

10. The process of recovering 3-hydroxypropionic acid of claim 1,
    wherein the crystal of 3-hydroxypropionate has a radioactive isotope content of 20 pMC (percent modern carbon) or more and a biocarbon content of 20 wt% or more as measured by ASTM D6866-21 standard.

11. The process of recovering 3-hydroxypropionic acid of claim 1,
    wherein the crystal of 3-hydroxypropionate has an interatomic spacing (d value) of 1.00 Å or more and 15.00 Å or less in a crystal derived from X-ray diffraction (XRD) analysis.

12. The process of recovering 3-hydroxypropionic acid of claim 1,
    wherein the crystal of 3-hydroxypropionate shows a peak between crystal lattices in a 2θ range of 8 to 22° in an X-ray diffraction (XRD) analysis.

13. The process of recovering 3-hydroxypropionic acid of claim 1,
    wherein the crystal of 3-hydroxypropionate has a glass transition temperature of -55 °C or more and -30 °C or less in a differential scanning calorimetry (DSC) analysis.

14. The process of recovering 3-hydroxypropionic acid of claim 1,
    wherein the crystal of 3-hydroxypropionate has a melting point of 30 °C or more and 170 °C or less in a differential scanning calorimetry (DSC) analysis.

15. The process of recovering 3-hydroxypropionic acid of claim 1,
    wherein the crystal of 3-hydroxypropionate has a crystallization temperature of 25 °C or more and 170 °C or less in a differential scanning calorimetry (DSC) analysis.

16. The process of recovering 3-hydroxypropionic acid of claim 1,
    wherein the crystal of 3-hydroxypropionate has a purity of 70% or more.

17. The process of recovering 3-hydroxypropionic acid of claim 1, further comprising the steps of:

producing a fermented solution of 3-hydroxypropionic acid by fermenting a strain having a 3-hydroxypropionic acid-producing capacity; and

forming a concentrate containing the 3-hydroxypropionic acid in an amount of 300 g/L or more by concentrating the fermented solution.

18. The process of recovering 3-hydroxypropionic acid of claim 17, further comprising the step of:

after the production of the fermented solution of 3-hydroxypropionic acid,
removing cells from the fermented solution; or
filtering or purifying the fermented solution.

19. The process of recovering 3-hydroxypropionic acid of claim 17,
wherein in the forming of the concentrate, the concentration is performed by evaporating the fermented solution.

20. The process of recovering 3-hydroxypropionic acid of claim 1,
wherein a 3-hydroxypropionic acid recovery rate is 40% or more.

21. A crystal of 3-hydroxypropionate of Structural formula 1 or Structural formula 2:

[Structural formula 1]     $Cation(3HP)_n$

[Structural formula 2]     $Cation(3HP)_n \cdot mH_2O$

wherein the Cation is a cation,

the 3HP is 3-hydroxypropionic acid bonding to the cation;
the n is a number of 3HPs bonding to the cation, which is an integer of 1 or more; and
the m is a number of water molecules in a hydrate, which is an integer of 1 or more.

22. The crystal of 3-hydroxypropionate of claim 21,
wherein the crystal of 3-hydroxypropionate has a particle size distribution $D_{50}$ of 20 $\mu$m or more and 90 $\mu$m or less, and $(D_{90}-D_{10})/D_{50}$ of 1.00 or more and 3.00 or less.

23. The crystal of 3-hydroxypropionate of claim 21,
wherein the crystal of 3-hydroxypropionate has an average aspect ratio (LW ratio; length to width ratio) of 0.50 or more and 3.00 or less.

24. The crystal of 3-hydroxypropionate of claim 21,
wherein the crystal of 3-hydroxypropionate has a radioactive isotope content of 20 pMC (percent modern carbon) or more and a biocarbon content of 20 wt% or more as measured by ASTM D6866-21 standard.

25. The crystal of 3-hydroxypropionate of claim 21,
wherein the crystal of 3-hydroxypropionate has an interatomic spacing (d value) of 1.00 Å or more and 15.00 Å or less in a crystal derived from X-ray diffraction (XRD) analysis.

26. The crystal of 3-hydroxypropionate of claim 21,
wherein the crystal of 3-hydroxypropionate shows a peak between crystal lattices in a $2\theta$ range of 8 to 22° in an X-ray diffraction (XRD) analysis.

27. The crystal of 3-hydroxypropionate of claim 21,
wherein the crystal of 3-hydroxypropionate has a glass transition temperature of -55 °C or more and -30 °C or less in a differential scanning calorimetry (DSC) analysis.

28. The crystal of 3-hydroxypropionate of claim 21,
wherein the crystal of 3-hydroxypropionate has a melting point of 30 °C or more and 170 °C or less in a differential scanning calorimetry (DSC) analysis.

29. The crystal of 3-hydroxypropionate of claim 21,

wherein the 3-hydroxypropionate contained in the crystal of the 3-hydroxypropionate has a purity of 70% or more.

30. A crystal of 3-hydroxypropionate,

comprising an alkali metal salt of 3-hydroxypropionic acid or a hydrate thereof,
wherein a content of biocarbon is 20 wt% or more as measured by ASTM D6866-21 standard.

31. The crystal of 3-hydroxypropionate of claim 30,
wherein the alkali metal salt of 3-hydroxypropionic acid is a calcium salt or magnesium salt of 3-hydroxypropionic acid.

32. The crystal of 3-hydroxypropionate of claim 30,
wherein the crystal of 3-hydroxypropionate has a mean diameter of volume distribution of 30 $\mu$m or more and 100 $\mu$m or less, a mean diameter of number distribution of 1 $\mu$m or more and 30 $\mu$m or less, and a mean diameter of area distribution of 10 $\mu$m or more and 70 $\mu$m or less.

33. The crystal of 3-hydroxypropionate of claim 30,
wherein the crystal of 3-hydroxypropionate has an average aspect ratio (LW ratio; length to width ratio) of 0.50 or more and 3.00 or less.

**FIG. 1**

EP 4 148 037 A1

FIG. 2

FIG. 3

**FIG. 4**

EP 4 148 037 A1

【FIG. 5】

【FIG. 6】

## INTERNATIONAL SEARCH REPORT

| International application No. |
| --- |
| **PCT/KR2022/000673** |

| A. | CLASSIFICATION OF SUBJECT MATTER |
| --- | --- |

**C07C 51/43**(2006.01)i; **C07C 51/50**(2006.01)i; **C07C 59/01**(2006.01)i; **C12P 7/42**(2006.01)i; **C12N 15/70**(2006.01)i; **C12N 9/88**(2006.01)i; **C12N 9/02**(2006.01)i; **C12N 9/10**(2006.01)i

According to International Patent Classification (IPC) or to both national classification and IPC

| B. | FIELDS SEARCHED |
| --- | --- |

Minimum documentation searched (classification system followed by classification symbols)

C07C 51/43(2006.01); B01D 9/00(2006.01); B01D 9/04(2006.01); C07C 51/42(2006.01); C12P 7/46(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), Google & & keywords: 3-하이드록시프로피온산(3-hydroxypropionic acid), 알칼리금속염 (alkali metal salt), 결정(crystal), 수산화칼슘(calcium hydroxide), 수산화마그네슘(magnesium hydroxide), 농축(concentrat ion), 분리(separation), 회수(recovery)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| Y | URBANUS, J. et al. Intensified crystallization in complex media: Heuristics for crystallization of platform chemicals. Chemical engineering science. 2012, vol. 77, pp. 18-25. <br> See abstract; pages 21 and 22; and tables 1-3. | 1-33 |
| Y | MCDONALD, M. A. et al. Reactive crystallization: a review. Reaction Chemistry & Engineering. 2021 ( publication date: 16 November 2020), vol. 6, no. 3, pp. 364-400 (inner pp. 1-37). <br> See inner pages 12 and 13. | 1-33 |
| Y | RAMACHANDRAN, S. et al. Gluconic acid: properties, applications and microbial production. Food Technology & Biotechnology. 2006, vol. 44, no. 2, pp. 185-195. <br> See abstract; and page 192. | 1-33 |
| A | US 5168055 A (DATTA, R. et al.) 01 December 1992 (1992-12-01) <br> See entire document. | 1-33 |

| ☑ Further documents are listed in the continuation of Box C. | ☑ See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **09 May 2022** | **09 May 2022** |

| Name and mailing address of the ISA/KR | Authorized officer |
| --- | --- |
| **Korean Intellectual Property Office** <br> **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

## INTERNATIONAL SEARCH REPORT

| International application No. |
| --- |
| **PCT/KR2022/000673** |

### C.    DOCUMENTS CONSIDERED TO BE RELEVANT

| Category* | Citation of document, with indication, where appropriate, of the relevant passages | Relevant to claim No. |
| --- | --- | --- |
| A | WO 2015-166098 A1 (NEDERLANDSE ORGANISATIE VOOR TOEGEPAST-NATUURWETENSCHAPPELIJK ONDERZOEK TNO) 05 November 2015 (2015-11-05)<br>See entire document. | 1-33 |

Form PCT/ISA/210 (second sheet) (July 2019)

INTERNATIONAL SEARCH REPORT
Information on patent family members

International application No.

**PCT/KR2022/000673**

| Patent document cited in search report | | | Publication date (day/month/year) | Patent family member(s) | | | Publication date (day/month/year) |
|---|---|---|---|---|---|---|---|
| US | 5168055 | A | 01 December 1992 | CA | 1338905 | C | 11 February 1997 |
| | | | | EP | 0405707 | A1 | 02 January 1991 |
| | | | | EP | 0405707 | B1 | 14 December 1994 |
| | | | | JP | 03-030685 | A | 08 February 1991 |
| WO | 2015-166098 | A1 | 05 November 2015 | CA | 2947064 | A1 | 05 November 2015 |
| | | | | EP | 3137184 | A1 | 08 March 2017 |
| | | | | US | 10265641 | B2 | 23 April 2019 |
| | | | | US | 2017-0050119 | A1 | 23 February 2017 |

Form PCT/ISA/210 (patent family annex) (July 2019)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 2022000673 W
- KR 1020210006246
- KR 1020210119997
- KR 1020200051375